# FASCICULE DE BREVET EUROPEEN

(11) **EP 2 037 806 B1**
(45) Date de publication et mention de la délivrance du brevet: **21.08.2019**
(21) Numéro de dépôt: 07803853.6
(22) Date de dépôt: 05.07.2007
(51) Int. Cl.: A61B 5/0476, A61B 5/0488, A61M 16/00

(54) **DISPOSITIF DE DÉTECTION D'UN RÉGLAGE INAPPROPRIÉ D'UNE MACHINE D'ASSISTANCE VENTILATOIRE UTILISÉE SUR UN MAMMIFÈRE**
VORRICHTUNG ZUR ERKENNUNG EINER UNGEEIGNETEN EINSTELLUNG EINER AN EINEM SÄUGETIER VERWENDETEN BEATMUNGSMASCHINE
DEVICE FOR DETECTING THE IMPROPER ADJUSTMENT OF A VENTILATORY SUPPORT MACHINE USED ON A MAMMAL

(30) Priorité: 10.07.2006 FR 0606261
(43) Date de publication de la demande: 25.03.2009
(73) Titulaire: Sorbonne Université, 75006 Paris (FR)
(72) Inventeur: SIMILOWSKI, Thomas, 92130 Issy-les-moulineaux (FR); RAUX, Mathieu, 63000 Clermont-Ferrand (FR); STRAUS, Christian, 78280 Guyancourt (FR); RAY, Patrick, 92290 Chatenay Malabry (FR)
(74) Mandataire: Lavoix
(86) Numéro de dépôt international: PCT/FR2007/001148
(87) Numéro de publication internationale: WO 2008/006963

(56) Documents cités:
- WO-A-99/43374
- US-A- 5 520 192
- US-A- 6 015 388
- US-B1- 6 317 627
- US-B1- 6 355 003
- US-B1- 6 390 091
- US-B1- 6 397 845
- US-B1- 6 411 843

## Description

La présente invention concerne un dispositif de détection d'un réglage inapproprié d'une machine d'assistance ventilatoire utilisée sur un mammifère.

Certaines personnes souffrent d'une défaillance respiratoire aiguë, consécutive, par exemple, à une pneumonie, un oedème pulmonaire ou une surinfection de maladies respiratoires chroniques. Une assistance ventilatoire mécanique peut s'avérer nécessaire. Les machines d'assistance ventilatoire ou ventilateurs comportent des moyens de détection d'une inspiration par le patient et des moyens pour aider le patient à l'inspiration en augmentant le flux d'air ou la pression de l'air recueilli par le patient.

Ainsi, l'assistance consiste en la fourniture d'un volume prédéterminé de gaz ou en une pressurisation des voies aériennes. Dans les deux cas, divers réglages permettent d'adapter le flux de gaz aux besoins du patient. La machine d'assistance doit donc être adaptée au comportement ventilatoire du patient afin que la relation qui les unit soit "harmonieuse", c'est-à-dire que le patient soit dans un état de confort physique satisfaisant, le patient n'éprouvant, lors de l'assistance, aucune gêne respiratoire. Avec des réglages inadaptés, par exemple lorsque le flux d'air est trop important ou, au contraire, trop faible, le patient peut être dans une situation inconfortable, voire être dans une situation de détresse respiratoire.

Pour détecter une telle dysharmonie entre le patient et la machine, différents moyens ont été utilisés. En particulier, il est connu de simplement interroger le patient. Toutefois, cela n'est pas possible lorsque le patient est endormi ou dans le coma.

Il est connu également d'observer l'activité ventilatoire, notamment la fréquence et l'utilisation des différents groupes musculaires respiratoires.

Il est également recouru à l'observation de la coordination entre les mouvements respiratoires et la réponse du ventilateur pour détecter un asynchronisme ou des efforts de déclenchement inefficaces.

Enfin, il est connu de mesurer des éléments physiologiques indirects permettant de qualifier l'activité ventilatoire et de détecter, le cas échéant, sa nature désynchronisée. Ces éléments physiologiques indirects sont par exemple la pression d'occlusion, la morphologie des courbes de pression des voies aériennes, le travail ventilatoire.

En pratique, ces moyens s'avèrent délicats à utiliser et constituent tous des témoins indirects des sensations que peut éprouver le patient.

L'invention a pour but de proposer un dispositif permettant de détecter un réglage défaillant de la machine de ventilation qui soit fiable.

Le document US-B1-6397845 décrit un dispositif qui, à l'aide de variables physiologiques d'entrée, telles qu'un électromyogramme, un électroencéphalogramme, la position du patient ou sa respiration, détermine l'état de sommeil du patient, puis modifie en fonction de l'état de sommeil la pression de gaz délivrée par une machine d'assistance respiratoire.

A cet effet, l'invention a pour objet un dispositif de détection d'un réglage inapproprié d'une machine d'assistance respiratoire utilisée sur un mammifère selon la revendication 1.

Ainsi, l'invention permet de détecter le réglage inapproprié à partir d'une activité neurologique ou neuromusculaire anormale chez un patient placé sous assistance ventilatoire mécanique. Une telle manière de faire permet une détection fiable, c'est-à-dire obtenue quasi directement des sensations du patient.

Suivant des modes particuliers de réalisation, le dispositif comporte l'une ou plusieurs des caractéristiques énoncées dans les revendications 2 à 15.

L'invention a également pour objet une installation d'assistance ventilatoire selon la revendication 16.

L'invention a également pour objet un procédé de détection d'un réglage inapproprié d'une machine d'assistance ventilatoire utilisée sur un mammifère selon la revendication 17.

L'invention a également pour objet un programme d'ordinateur selon la revendication 18.

L'invention sera mieux comprise à la lecture de la description qui va suivre, donnée uniquement à titre d'exemple et faite en se référant aux dessins, sur lesquels :
- la figure 1 est une vue schématique d'une installation d'assistance ventilatoire mise en oeuvre sur un patient ;
- la figure 2 est une vue en perspective de trois-quarts arrière de la tête d'un être humain montrant un exemple d'implantation des électrodes ;
- la figure 3 est un ensemble de courbes explicitant le moyennage point à point effectué sur un ensemble de cycles respiratoires du signal électroencéphalographique recueilli ;
- la figure 4 est un exemple d'ensemble de courbes montrant l'activité encéphalographique détectée et l'activité électromyographique du muscle scalène d'un être humain placé sous assistance ventilatoire ;
- la figure 5 est une vue identique à celle de la figure 4 en cas de dysharmonie est un exemple d'ensemble de courbes montrant l'activité encéphalographique détectée et l'activité électromyographique du muscle scalène d'un être humain placé sous assistance ventilatoire ;
- la figure 6 est une vue identique à celle de la figure 1 d'une variante de réalisation d'une installation selon l'invention ;
- la figure 7 est un exemple d'ensemble de courbes montrant l'activité ventilatoire (en haut) et l'activité électromyographique (en bas) pour plusieurs cycles respiratoires ; et
- la figure 8 est une courbe montrant l'activité ventilatoire (en haut) et l'enveloppe moyenne de l'activité électromyographique (en bas).

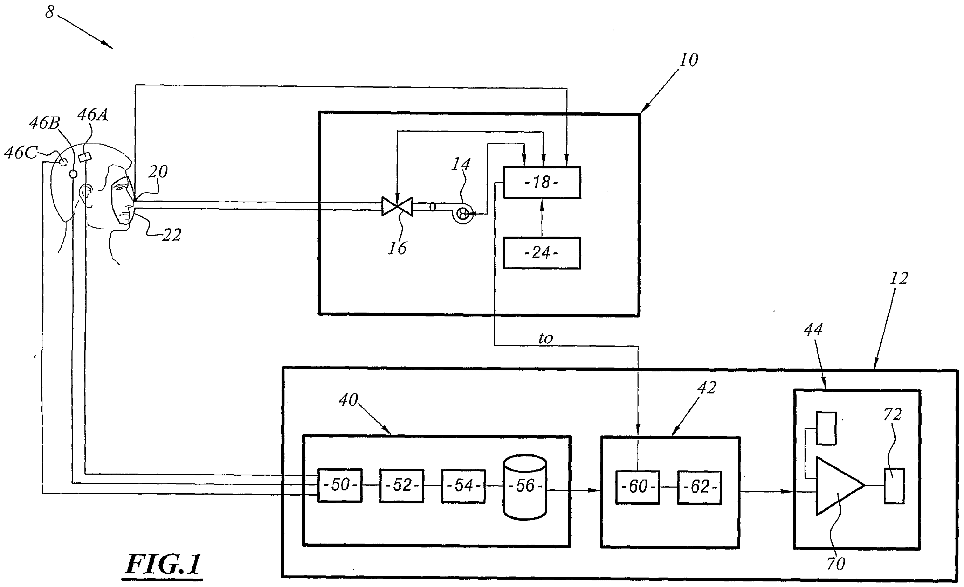

Sur la figure 1 est illustrée un exemple d'une installation 8 d'assistance ventilatoire utilisant un dispositif selon l'invention.

Cette installation 8 comporte une machine d'assistance ventilatoire mécanique 10 et un dispositif 12 de détection d'un réglage défaillant de la machine d'assistance 10.

La machine 10 comporte, comme connu en soi, une turbine 14 propre à fournir un flux d'air à un patient à un débit déterminé et avec une pression donnée. En sortie de la turbine 14 est prévue une vanne 16 permettant de fournir ou non l'air au patient sous pression produit par la turbine 14. La turbine 14 et la vanne 16 sont reliées à une unité de pilotage 18 elle-même connectée à un capteur de dépression propre à détecter une aspiration du patient.

La turbine 14 est reliée, en aval de la vanne 16, à un masque 22 propre à être appliqué sur les voies respiratoires supérieures du patient. Le capteur de dépression 20 est monté par exemple dans le masque 22 du patient.

En variante, le masque 22 peut être remplacé par une sonde endotrachéale.

L'unité de pilotage 18 est reliée à une unité de réglage 24 propre à modifier les paramètres de fonctionnement de la machine 18, et en particulier le débit imposé par la turbine 14, la pression du flux, les instants de basculement de la vanne 16, et tout autre paramètre comme connu dans l'état de la technique.

Par ailleurs, l'unité de pilotage 18 comporte une sortie propre à fournir un signal de déclenchement respiratoire tₒ représentatif d'un début d'inspiration du patient.

Le dispositif 12 de détection d'un réglage défaillant comporte une unité 40 de mesure d'un signal neurophysiologique représentatif d'une inspiration et propre à fournir un signal neurophysiologique en fonction du temps.

Elle comporte en outre une unité 42 de traitement connectée à l'unité de mesure 40 pour recevoir le signal neurophysiologique. Cette unité de traitement 42 comporte une entrée pour la réception du signal de déclenchement respiratoire t₀, cette entrée étant connectée à la sortie correspondante de la machine 10.

Le dispositif 12 comporte en outre des moyens 44 de mise à disposition d'un praticien d'une information représentative d'un réglage défaillant de la machine 10.

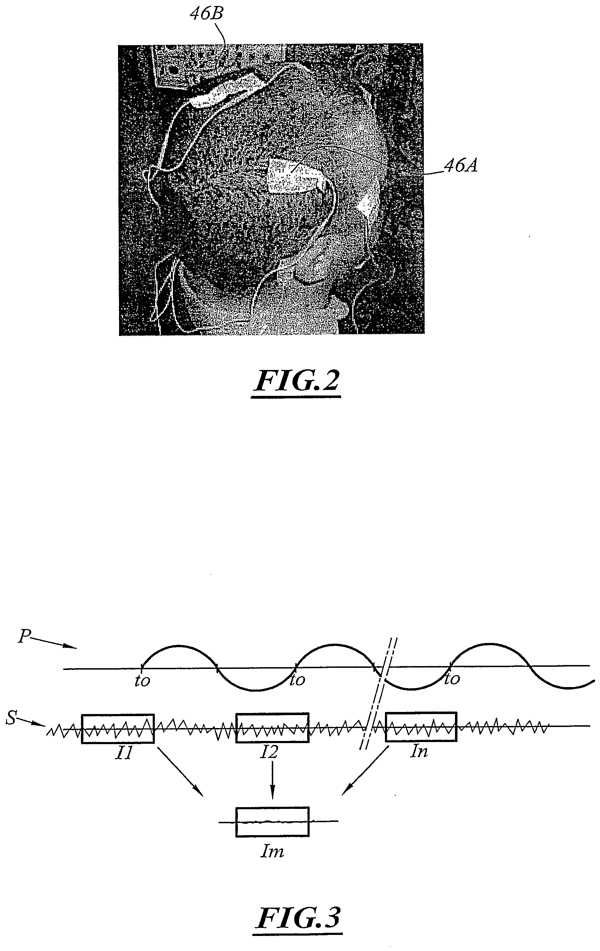

Selon un premier mode de réalisation, les moyens 40 de mesure d'un signal neurophysiologique sont formés d'un électroencéphalographe. Celui-ci comporte, par exemple, trois électrodes 46A, 46B, 46C disposées sur le crâne du patient et notamment au niveau de l'aire motrice supplémentaire, c'est-à-dire du cortex prémoteur. Plus précisément, et de préférence, les électrodes 46A, 46B, 46C sont placées au niveau des dérivations C3-A+, C4-A+ et Cz-A+ du scalp comme illustré sur la figure 2, ces positions étant déterminées dans le repère 10-20 du système international.

Comme connu en soi, l'électroencéphalographe comporte des moyens 50 de recueil du signal, des moyens 52 de filtrage et d'amplification, ainsi que des moyens 54 d'échantillonnage afin de numériser le signal, par exemple avec une fréquence d'échantillonnage de 2000 Hz. Il comporte en outre des moyens 56 de stockage informatiques des valeurs échantillonnées avec leur instant d'échantillonnage correspondant.

Les moyens de traitement 42 comportent des moyens 60 de rétromoyennage des valeurs échantillonnées mémorisées sur plusieurs cycles respiratoires. Ils sont formés par exemple d'un micro-ordinateur mettant en oeuvre un programme adapté. Plus précisément, et comme illustré sur la figure 3, les moyens de traitement sont propres à effectuer la moyenne arithmétique point à point entre différents intervalles de temps successifs I₁, I₂, Iₙ du signal enregistré, chaque intervalle de temps I₁, I₂, Iₙ correspondant à un cycle respiratoire. Chaque intervalle de temps I₁, I₂, Iₙ est définit par rapport à l'instant de déclenchement respiratoire tₒ du cycle respiratoire correspondant et est calé sur cet instant tₒ. On entend par cycle respiratoire l'intervalle de temps constitué d'une expiration et d'une inspiration complètes.

Sur cette figure 3, la courbe P illustre la pression mesurée par le capteur 20 alors que la courbe S illustre le signal électroencéphalographique recueilli aux mêmes instants.

Les intervalles de temps successifs I₁, I₂, Iₙ sur lesquels est effectué le moyennage des signaux sont illustrés.

Ils sont chacun calé sur l'instant tₒ de déclenchement respiratoire et ont tous la même durée.

Le signal moyen noté Iₘ est obtenu par moyennage des signaux des intervalles I₁, I₂, Iₙ. Un tel signal Iₘ moyen est déterminé pour chacune des trois électrodes 46A, 46B, 46C. La moyenne est de préférence réalisée sur un nombre n de cycles successifs supérieur à 10 et par exemple compris entre 50 et 100 et avantageusement égal à 80.

La durée des intervalles de moyennage I₁, I₂, Iₙ est au maximum égale à la durée d'un cycle respiratoire. Elle est de préférence comprise entre 2 s et 4 s et de préférence sensiblement égale à trois secondes.

L'intervalle de moyennage I₁, I₂, Iₙ comprend l'instant de déclenchement respiratoire tₒ. Cet intervalle s'étend avantageusement pour plus de la moitié en avance par rapport à l'instant de déclenchement respiratoire tₒ. De préférence, il s'étend pour plus des deux tiers en avance par rapport à l'instant de déclenchement tₒ. Plus précisément, le début de l'intervalle est antérieur de 1,5 s à 3,5 s à l'instant de déclenchement respiratoire tₒ. De préférence, le début est antérieur sensiblement à 2,5 s.

La fin de l'intervalle est postérieure à l'instant de déclenchement tₒ respiratoire d'un retard compris entre 0,2 s et 0,7 s et de préférence sensiblement égal à 0,5 s.

Les moyens de traitement 42 comportent en outre des moyens de calcul de la pente du signal électroencéphalographique moyenné observé antérieurement à l'instant de déclenchement respiratoire tₒ.

Les moyens 44 de mise en évidence d'un réglage défaillant comportent des moyens 70 de comparaison de la pente du signal moyenné Iₘ à une valeur de référence et des moyens 72 de déclenchement d'un indicateur tel qu'un voyant lumineux, lorsque la valeur de la pente est supérieure à une valeur de référence.

En pratique, cette valeur de référence est par exemple prise égale à zéro.

Outre l'indicateur, les moyens 44 de mise en évidence comportent avantageusement des moyens de stockage et d'affichage des signaux moyennés Iₘ et des valeurs de la pente calculée pour chaque signal Iₘ.

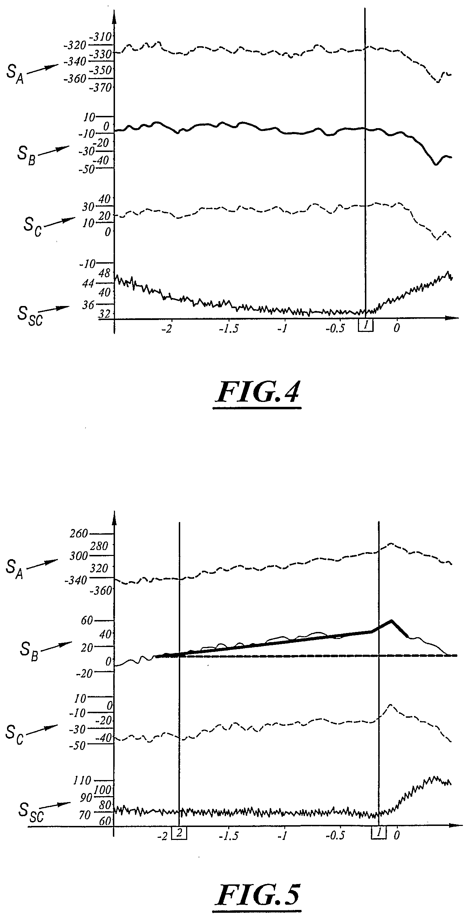

Sur les figures 4 et 5 est représenté un exemple de trois valeurs de signaux moyennées S_{A}, S_{B}, S_{C} obtenues depuis les capteurs 46A, 46B, 46C sur un patient en harmonie avec le ventilateur sur la figure 4 et un patient en dysharmonie sur la figure 5.

Il a été constaté que, en cas d'harmonie entre le patient et la machine d'assistance, aucun potentiel prémoteur n'est constaté avant l'instant de déclenchement respiratoire tₒ, le potentiel prémoteur étant le potentiel électroencéphalographique mesuré dans la phase précédant immédiatement l'instant de déclenchement respiratoire tₒ.

En revanche, en cas de dysharmonie, le potentiel prémoteur augmente progressivement avant l'instant de déclenchement respiratoire tₒ. Ainsi, il est fait l'hypothèse que, lors d'une dysharmonie, un potentiel prémoteur traduisant la préparation corticale du mouvement s'établit avant l'instant de déclenchement tₒ.

Le dispositif étant apte à détecter ce potentiel prémoteur, celui-ci est susceptible de détecter une dysharmonie entre la machine respiratoire et le patient.

A partir de l'information fournie par les moyens de mise en évidence, le praticien est susceptible de modifier les réglages de la machine d'assistance 10 depuis l'unité de réglage 24. En variante, une boucle de régulation est établie entre les moyens de mise en évidence et la machine d'assistance 10, les réglages étant automatiquement modifiés en fonction de la valeur de la pente du potentiel prémoteur détectée.

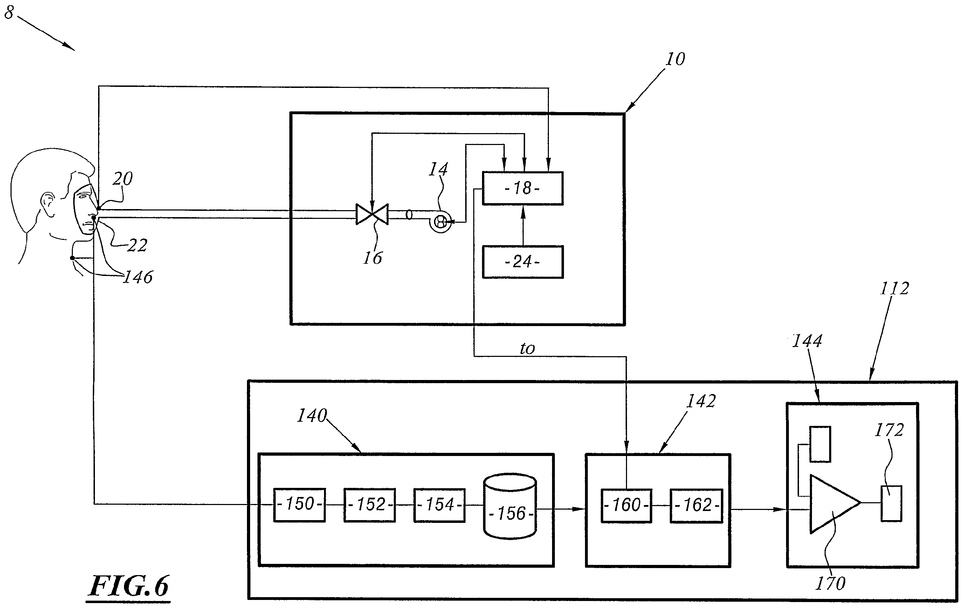

Suivant un autre mode de réalisation du dispositif noté 112 illustré sur la figure 6, les moyens de mesure d'un signal neurophysiologique sont remplacés par un électromyographe 140. Celui-ci comporte une ou plusieurs électrodes 146 propres à être placées sur les muscles respiratoires, c'est-à-dire activés par la respiration, par exemple du cou ou du visage d'un patient (en particulier les ailes du nez). Ces électrodes sont susceptibles de détecter les signaux électriques de ces muscles. Ces électrodes sont placées par exemple, suivant un premier mode de réalisation, sur le muscle scalène ou, suivant un second mode de réalisation, sur le muscle alaenasi, commandant l'ouverture des narines. Dans le premier cas, l'électrode 146 est avantageusement placée directement dans le masque 22 assurant l'alimentation en air du patient. Le signal inférieur Ssc des figures 4 et 5 représente l'activité électromyographique enregistrée du muscle scalène.

Comme précédemment, le dispositif 112 comporte une unité 142 de traitement reliée à l'électromyographe 140 et des moyens 144 de mise à disposition d'une information représentative d'un réglage défaillant.

Comme connu en soi, l'électromyographe 140 comporte des moyens 150 de recueil du signal, des moyens de filtrage et d'amplification 152 et des moyens d'échantillonnage 154 ayant par exemple une fréquence de 10 000 Hz, le filtrage étant effectué entre 20 Hz et 3 kHz. Comme précédemment, l'électromyographe 140 comporte des moyens 156 de stockage des valeurs échantillonnées avec leurs instants d'échantillonnage correspondant.

Les moyens de traitement 142 comportent des moyens de rétromoyennage 160 des valeurs échantillonnées.

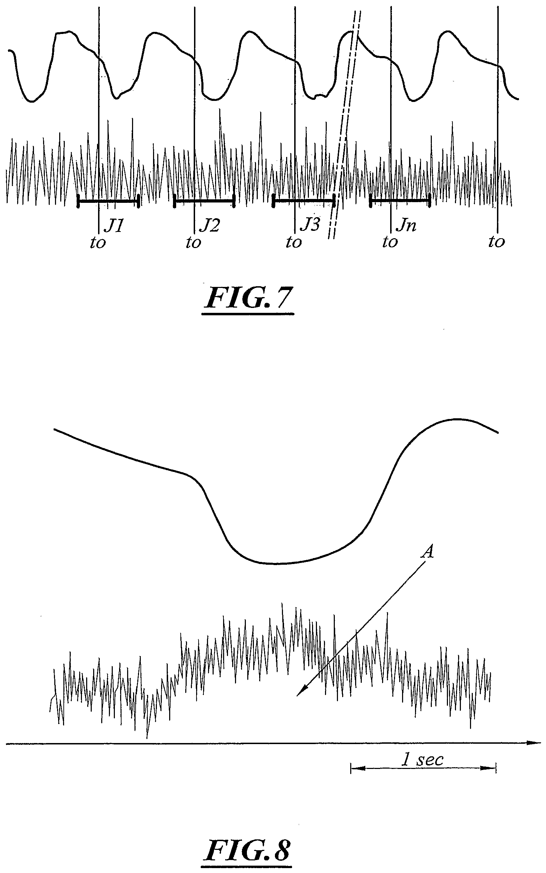

En référence à la figure 7, dans ce mode de réalisation, les moyens de traitement 142 sont propres à effectuer le calcul de la racine carrée du signal, puis la moyenne arithmétique point à point de cette racine carrée pour un nombre n de cycles prédéterminés sur des intervalles de temps J₁, J₂, J₃,..., Jₙ définis par rapport à l'instant de déclenchement respiratoire tₒ de chaque cycle et calés sur cet instant.

En effet, le signal étant dans ce mode de réalisation symétrique par rapport à l'axe des abscisses, il est nécessaire de le rendre asymétrique en l'élevant au carré afin de calculer une moyenne non nulle.

Avantageusement, la moyenne de la racine carrée est réalisée pour chaque intervalle de temps J₁, J₂, J₃,..., Jₙ sur une fenêtre temporelle mobile, de préférence de durée de 1 milliseconde, parcourant ledit intervalle pour obtenir une enveloppe pour chaque intervalle.

La durée de l'intervalle de moyennage J₁, J₂, J₃,..., Jₙ est au maximum égale à la durée d'un cycle respiratoire. Elle est de préférence comprise entre 2 s et 4 s et de préférence sensiblement égale à 3 s. L'intervalle comprend l'instant de déclenchement respiratoire tₒ. Cet intervalle s'étend avantageusement pour plus de la moitié et avantageusement pour plus des trois-quarts après l'instant de déclenchement respiratoire tₒ. Plus précisément, le début de l'intervalle est antérieur de 0,5 s à 1,5 s à l'instant de déclenchement respiratoire tₒ. De préférence, le début est antérieur sensiblement à une seconde.

La fin de l'intervalle est postérieure à l'instant de déclenchement respiratoire tₒ d'un retard compris entre 1 et 3 secondes et de préférence sensiblement égale à 2 secondes.

L'unité de traitement 142 comporte en outre des moyens 162 de calcul d'une enveloppe moyenne représentative de l'allure des électromyogrammes des n cycles précédents Cette enveloppe moyenne est illustrée sur la figure 8 avec l'échelle (1 sec) indiquée. On moyenne à cet effet les enveloppes des différents intervalles J₁, J₂, J₃,..., Jₙ pour obtenir l'enveloppe moyenne. L'enveloppe moyenne est de préférence réalisée pour un nombre n de cycles, c'est-à-dire d'intervalles J₁, J₂, J₃,..., Jₙ, supérieur à 10 et compris entre 50 et 100 et avantageusement égal à 80.

Les moyens 144 de mise en évidence d'un réglage défaillant comporte des moyens 170 de détection d'une évolution temporelle de la valeur de l'enveloppe moyenne calculée.

A cet effet, et pour différents instants de mesure, l'enveloppe moyenne est mémorisée. Ces moyens de détection 170 sont propres à comparer les différentes valeurs des enveloppes moyennes mémorisées entre les intervalles J₁, J₂, J₃,..., Jₙ.

De préférence, une enveloppe moyenne est calculée à une fréquence de 2 à 15 fois par heure et de préférence sensiblement égale à 5 fois par heure

En variante, l'enveloppe moyenne est calculée sur demande, par exemple de la part du personnel hospitalier.

En l'absence d'évolution temporelle dans la valeur de l'enveloppe, les moyens 172 ne déclenchent pas l'indicateur de réglage défaillant.

En revanche, en cas d'évolution temporelle de cette valeur, et notamment d'augmentation de l'intégrale de l'enveloppe moyenne (référence A sur la figure 8), les moyens de déclenchement sont adaptés pour commander le déclenchement d'un indicateur.

Il a été constaté qu'en cas d'harmonie, la valeur de l'intégrale reste constante. En revanche, en cas de désharmonie, l'intégrale tend à augmenter.

## Revendications

1. Dispositif (12 ; 112) de détection d'un réglage inapproprié d'une machine d'assistance ventilatoire utilisée sur un mammifère, **caractérisé en ce qu'**il comporte :
- des moyens (40 ; 140) de mesure, en fonction du temps, d'un signal neurophysiologique mis en oeuvre dans le processus respiratoire du mammifère pour au moins deux cycles respiratoires successifs, chaque cycle respiratoire comportant un instant de déclenchement respiratoire ;
- une entrée pour la réception d'un signal de déclenchement respiratoire (tₒ) différent du signal neurophysiologique ;
- des moyens (42 ; 142) de traitement des signaux neurophysiologiques, configurés pour traiter les signaux neurophysiologiques, pour chaque instant de déclenchement respiratoire (tₒ), sur un intervalle de temps comprenant l'instant de déclenchement respiratoire (tₒ) et s'étendant en avance de l'instant de déclenchement respiratoire (tₒ) ; et
- des moyens (44 ; 144) de mise en évidence d'un réglage inapproprié du ventilateur à partir desdits signaux traités.

2. Dispositif (12 ; 112) selon la revendication 1, dans lequel les moyens de traitement (42 ; 142) comportent des moyens (60 ; 160) de rétro-moyennage point à point des signaux neurophysiologiques mesurés pour l'ensemble des cycles sur un même intervalle de temps déterminé.

3. Dispositif (12) selon l'une quelconque des revendications précédentes, **caractérisé en ce que** les moyens de mesure comprennent un électroencéphalographe (40) et sont propres à mesurer un éventuel potentiel pré-moteur du mammifère précédent l'instant de déclenchement respiratoire (tₒ).

4. Dispositif (12) selon la revendication 3, dans lequel les moyens de traitements sont configurés pour calculer la pente du signal électroencéphalographique immédiatement avant l'instant de déclenchement respiratoire (tₒ).

5. Dispositif selon la revendication 4, dans lequel les moyens (44) de mise en évidence comportent des moyens (70) de comparaison de la pente avec une valeur de référence.

6. Dispositif (12) selon la revendication 5, dans lequel la valeur de référence est prise égale à zéro.

7. Dispositif selon la revendication 5 ou 6, dans lequel les moyens (44) de mise en évidence d'un réglage inapproprié comportent des moyens (72) de déclenchement d'un indicateur, lorsque la valeur de la pente est supérieure à la valeur de référence.

8. Dispositif selon la revendication 7, dans lequel l'indicateur est un voyant lumineux.

9. Dispositif (12) selon l'une quelconque des revendications 3 à 8, dans lequel ledit intervalle de temps s'étend pour plus de la moitié en avance par rapport à l'instant de déclenchement respiratoire (tₒ).

10. Dispositif (112) selon la revendication 1 ou 2, dans lequel les moyens de mesure comprennent un électromyographe (144) et sont propres à calculer l'intégrale d'un signal électromyographique sur ledit intervalle de temps (tₒ).

11. Dispositif (112) selon la revendication 10, dans lequel l'intervalle de temps s'étend pour plus de la moitié en retard par rapport à l'instant respiratoire (tₒ).

12. Dispositif (112) selon la revendication 10 ou 11, dans lequel les moyens de mise en évidence (144) comprennent des moyens (170) de détection d'une évolution temporelle de la moyenne de la racine carrée du signal électromyographique sur ledit intervalle de temps.

13. Dispositif selon l'une quelconque des revendications 1 à 12, lequel comprend un capteur propre à détecter une aspiration du patient, relié à l'entrée pour la réception d'un signal de déclenchement respiratoire (tₒ), ce capteur étant distinct des moyens (40 ; 140) de mesure du signal neurophysiologique.

14. Dispositif selon la revendication 13, dans lequel le capteur est un capteur de dépression.

15. Dispositif selon la revendication 13 ou 14, dans lequel le capteur est distinct des moyens de mesure (40 ; 140).

16. Installation (8) d'assistance ventilatoire comportant :
- une machine d'assistance ventilatoire (10) ; et
- un dispositif (12) de détection d'un réglage inapproprié selon l'une quelconque des revendications précédentes.

17. Procédé (12 ; 112) de détection d'un réglage inapproprié d'une machine d'assistance ventilatoire utilisée sur un mammifère, **caractérisé en ce qu'**il comporte les étapes de :
- réception d'une mesure, en fonction du temps, d'un signal neurophysiologique mis en oeuvre dans le processus respiratoire du mammifère pour au moins deux cycles respiratoires ; chaque cycle respiratoire comportant un instant de déclenchement respiratoire ;
- la réception d'un signal de déclenchement respiratoire (tₒ), différent du signal neurophysiologique ;
- le traitement des signaux neurophysiologiques, pour chaque instant de déclenchement respiratoire (tₒ), sur un intervalle de temps comprenant l'instant de déclenchement respiratoire (tₒ) et s'étendant en avance de l'instant de déclenchement respiratoire (tₒ) ; et
- mise en évidence d'un réglage inapproprié de la machine d'assistance ventilatoire à partir desdits signaux traités.

18. Produit programme d'ordinateur comportant des instructions qui, lorsque mis en oeuvre sur un calculateur associé à des moyens de mesure d'un signal neurophysiologique, met en oeuvre le procédé de la revendication 17.

## Patentansprüche

1. Vorrichtung (12, 112) zum Detektieren einer ungeeigneten Einstellung einer Atmungsunterstützungsmaschine, die bei einem Säugetier verwendet wird, **dadurch gekennzeichnet, dass** sie aufweist:
- Mittel (40, 140) zum Messen, in Abhängigkeit von der Zeit, eines neurophysiologischen Signals, das beim Atmungsvorgang des Säugetiers umgesetzt wird, für mindestens zwei aufeinanderfolgende Atemzyklen, wobei jeder Atemzyklus einen Atmungsauslösung-Zeitpunkt aufweist,
- einen Eingang für das Empfangen eines Atmungsauslösung-Signals (tₒ), das sich von dem neurophysiologischen Signal unterscheidet,
- Mittel (42, 142) zum Verarbeiten der neurophysiologischen Signale, die dazu eingerichtet sind, die neurophysiologischen Signale für jeden Atmungsauslösung-Zeitpunkt (tₒ) über ein Zeitintervall, welches den Atmungsauslösung-Zeitpunkt (tₒ) aufweist und sich vor dem Atmungsauslösung-Zeitpunkt (tₒ) erstreckt, zu verarbeiten, und
- Mittel (44, 144) zum Nachweisen einer ungeeigneten Einstellung des Lüfters ausgehend von den verarbeiteten Signalen.

2. Vorrichtung (12, 112) gemäß dem Anspruch 1, wobei die Mittel zum Verarbeiten (42, 142) Mittel (60, 160) zur Punktfür-Punkt-Rück-Mittelwertbildung der für die Gesamtheit der Zyklen über ein gleiches bestimmtes Zeitintervall gemessenen neurophysiologischen Signale aufweisen.

3. Vorrichtung (12) gemäß irgendeinem der vorhergehenden Ansprüche, **dadurch gekennzeichnet, dass** die Mittel zum Messen einen Elektroenzephalographen (40) aufweisen und in der Lage sind, ein mögliches prämotorisches Potential des Säugetiers zu messen, das dem Atmungsauslösung-Zeitpunkt (tₒ) vorausgeht.

4. Vorrichtung (12) gemäß dem Anspruch 3, wobei die Verarbeitungsmittel dazu eingerichtet sind, die Steigung des elektroenzephalographischen Signals unmittelbar vor dem Atmungsauslösung-Zeitpunkt (tₒ) zu berechnen.

5. Vorrichtung gemäß dem Anspruch 4, wobei die Mittel (44) zum Nachweisen Mittel (70) zum Vergleichen der Steigung mit einem Referenzwert aufweisen.

6. Vorrichtung (12) gemäß dem Anspruch 5, wobei der Referenzwert gleich Null angesetzt wird.

7. Vorrichtung gemäß dem Anspruch 5 oder 6, wobei die Mittel (44) zum Nachweisen einer ungeeigneten Einstellung Mittel (72) zum Auslösen eines Indikators aufweisen, wenn der Wert der Steigung größer als der Referenzwert ist.

8. Vorrichtung gemäß dem Anspruch 7, wobei der Indikator eine Leuchtanzeige ist.

9. Vorrichtung (12) gemäß irgendeinem der Ansprüche 3 bis 8, wobei sich das Zeitintervall für mehr als die Hälfte vor dem Atmungsauslösung-Zeitpunkt (tₒ) erstreckt.

10. Vorrichtung (112) gemäß dem Anspruch 1 oder 2, wobei die Mittel zum Messen einen Elektromyographen (144) aufweisen und in der Lage sind, das Integral eines elektromyographischen Signals über das Zeitintervall (tₒ) zu berechnen.

11. Vorrichtung (112) gemäß dem Anspruch 10, wobei sich das Zeitintervall für mehr als die Hälfte nach dem Atmungsauslösung-Zeitpunkt (tₒ) erstreckt.

12. Vorrichtung (112) gemäß dem Anspruch 10 oder 11, wobei die Mittel zum Nachweisen (144) Mittel (170) zum Detektieren einer zeitlichen Entwicklung des Mittelwerts der Quadratwurzel des elektromyographischen Signals über das Zeitintervall aufweisen.

13. Vorrichtung gemäß irgendeinem der Ansprüche 1 bis 12, wobei diese einen Sensor aufweist, der in der Lage ist, ein Atmen des Patienten zu detektieren, der verbunden ist mit dem Eingang zum Empfangen eines Atmungsauslösung-Signals (tₒ), wobei sich der Sensor von den Mitteln (40, 140) zum Messen des neurophysiologischen Signals unterscheidet.

14. Vorrichtung gemäß dem Anspruch 13, wobei der Sensor ein Unterdrucksensor ist.

15. Vorrichtung gemäß dem Anspruch 13 oder 14, wobei der Sensor sich von den Mitteln zum Messen (40, 140) unterscheidet.

16. Einrichtung (8) zur Atmungsunterstützung, aufweisend:
- eine Atmungsunterstützungsmaschine (10) und
- eine Vorrichtung (12) zum Detektieren einer ungeeigneten Einstellung gemäß irgendeinem der vorhergehenden Ansprüche.

17. Verfahren (12, 112) zum Detektieren einer ungeeigneten Einstellung einer Atmungsunterstützungsmaschine, die bei einem Säugetier verwendet wird, **dadurch gekennzeichnet, dass** es die folgenden Schritte aufweist:
- das Empfangen einer Messung, in Abhängigkeit von der Zeit, eines neurophysiologischen Signals, das beim Atmungsvorgang des Säugetiers umgesetzt wird, für mindestens zwei Atemzyklen, wobei jeder Atemzyklus einen Atmungsauslösung-Zeitpunkt aufweist,
- das Empfangen eines Atmungsauslösung-Signals (tₒ), das sich von dem neurophysiologischen Signal unterscheidet,
- das Verarbeiten der neurophysiologischen Signale für jeden Atmungsauslösung-Zeitpunkt (tₒ) über ein Zeitintervall, welches den Atmungsauslösung-Zeitpunkt (tₒ) aufweist und sich vor dem Atmungsauslösung-Zeitpunkt (tₒ) erstreckt, und
- das Nachweisen einer ungeeigneten Einstellung der Atmungsunterstützungsmaschine ausgehend von den verarbeiteten Signalen.

18. Computerprogrammprodukt, das Befehle aufweist, das, wenn es auf einem Rechner ausgeführt wird, der mit Mitteln zum Messen eines neurophysiologischen Signals verknüpft ist, das Verfahren gemäß dem Anspruch 17 durchführt.

## Claims

1. Device (12; 112) for detecting the improper adjustment of a ventilatory support machine used on a mammal, **characterized in that** it comprises:
- means (40; 140) for measuring, as a function of time, a neurophysiological signal involved in the respiratory process of the mammal for at least two successive respiratory cycles, each respiratory cycle comprising a respiratory initiation time;
- an input for receiving a respiratory initiation signal (tₒ) which is different from the neurophysiological signal;
- means (42; 142) for processing the neurophysiological signals, which means are configured for processing the neurophysiological signals for each respiratory initiation time (tₒ) over a period of time comprising the respiratory initiation time (tₒ) and starting before the respiratory initiation time (tₒ); and
- means (44; 144) for detecting an improper adjustment of the ventilator by means of said processed signals.

2. Device (12; 112) according to claim 1, wherein the processing means (42; 142) comprise means (60; 160) for back-averaging, point-to-point, the neurophysiological signals measured for all cycles over the same specified period of time.

3. Device (12) according to any one of the preceding claims, wherein the measuring means comprise an electroencephalograph (40) and are used to measure possible premotor potential of the mammal before the respiratory initiation time (tₒ).

4. Device (12) according to claim 3, wherein the processing means are configured for calculating the slope of the electroencephalographic signal immediately before the respiratory initiation time (tₒ).

5. Device according to claim 4, wherein the detection means (44) comprise means (70) for comparing the slope with a reference value.

6. Device (12) according to claim 5, wherein the reference value is equal to zero.

7. Device according to either claim 5 or claim 6, wherein the means (44) for detecting an improper adjustment comprise means (72) for triggering an indicator when the value of the slope is greater than the reference value.

8. Device according to claim 7, wherein the indicator is an indicator light.

9. Device (12) according to any one of claims 3 to 8, wherein more than half of said period of time elapses before the respiratory initiation time (tₒ).

10. Device (112) according to either claim 1 or claim 2, wherein the measuring means comprise an electromyograph (144) and are used to calculate the integral of an electromyographic signal over said period of time (tₒ).

11. Device (112) according to claim 10, wherein more than half of the period of time elapses after the respiratory time (tₒ).

12. Device (112) according to either claim 10 or claim 11, wherein the detection means (144) comprise means (170) for detecting a change over time in the root mean square of the electromyographic signal over said period of time.

13. Device according to any one of claims 1 to 12, wherein it comprises a sensor for detecting a patient's aspiration, connected to the input for receiving a respiratory initiation signal (tₒ), said sensor being separate from the means (40, 140) for measuring the neurophysiological signal.

14. Device according to claim 13, wherein the sensor is a load sensor.

15. Device according to either claim 13 or claim 14, wherein the sensor is separate from the measuring means (40; 140).

16. Ventilatory support assembly (8) comprising:
- a ventilatory support machine (10); and
- a device (12) for detecting improper adjustment according to any one of the preceding claims.

17. Method (12; 112) for detecting the improper adjustment of a ventilatory support machine used on a mammal, **characterized in that** it comprises the steps of:
- measuring, as a function of time, a neurophysiological signal involved in the respiratory process of the mammal for at least two respiratory cycles; each respiratory cycle comprising a respiratory initiation time;
- receiving a respiratory initiation signal (tₒ) which is different from the neurophysiological signal;
- processing the neurophysiological signals for each respiratory initiation time (tₒ) over a period of time comprising the respiratory initiation time (tₒ) and starting before the respiratory initiation time (tₒ); and
- detecting an improper adjustment of the ventilator by means of said processed signals.

18. Computer software package comprising instructions which, when said software package is loaded onto a computer connected to the means for measuring a neurophysiological signal, carries out the method of claim 17.
